# EUROPÄISCHE PATENTSCHRIFT

(11) **EP 4 115 811 B1**
(45) Veröffentlichungstag und Bekanntmachung des Hinweises auf die Patenterteilung: **27.08.2025**
(21) Anmeldenummer: 21183846.1
(22) Anmeldetag: 06.07.2021
(51) Int. Cl.: A61B 6/00, A61B 6/51

(54) **INTRAORAL-RÖNTGENSENSOR FÜR AUTOMATISCHE BELICHTUNGSKONTROLLE**
INTRAORAL X-RAY SENSOR FOR AUTOMATIC EXPOSURE CONTROL
CAPTEUR RADIOGRAPHIQUE INTRA-ORAL POUR LE CONTRÔLE AUTOMATIQUE DE L'EXPOSITION

(43) Veröffentlichungstag der Anmeldung: 11.01.2023
(73) Patentinhaber: DENTSPLY SIRONA Inc., York, PA 17401 (US); Sirona Dental Systems GmbH, 64625 Bensheim (DE)
(72) Erfinder: Schulze-Ganzlin, Ulrich, 64653 Lorsch (DE); Lindenberg, Kai, 64625 Bensheim (DE)
(74) Vertreter: Aldridge, Henry Alexander

(56) Entgegenhaltungen:
- EP-A1- 3 399 906
- US-A1- 2003 026 387

## Beschreibung

### TECHNISCHES GEBIET DER ERFINDUNG

Die vorliegende Erfindung bezieht sich auf ein intraorales Röntgensystem mit automatischen Belichtungskontrolle (AEC) Funktion, bestehend aus einem intraoralen Röntgensensor und einem dentalen Röntgengerät.

### HINTERGRUND DER ERFINDUNG

Die automatischen Belichtungskontrolle, nämlich die AEC (*automatic exposure control*)-Technik ist vorbekannt, auch im Dentalbereich für intraorales Röntgen mit digitalen Röntgenbildempfängern, siehe z.B. EP1859659A1 und US 6,898,268 B2.

Röntgenbilddetektoren haben für typische Belichtungssituationen in der intraoralen Röntgendiagnostik optimierten Dynamikumfang. Unterbelichtete Aufnahmen zeichnen sich durch erhöhtes Rauschen aus, überbelichtete Bereiche können aufgrund von Sättigungseffekten der Pixel das Signal nicht mehr auflösen. Mit Hilfe der AEC-Technik kann die Sättigungsgrenze durch Mehrfachbelichtungen und Aufsummierung der Einzelaufnahmen aufgelöst werden. Durch Senken der Sättigungsgrenze kann der Röntgenbildempfänger für niedrig-Belichtungen optimiert werden. Mit dieser Konstellation kann der Anwender indikationsabhängig die jeweils erforderliche Mindestbildqualität gezielt festlegen um die Patientendosis so niedrig wie möglich zu halten.

Mit einem Scout Shot kann die Belichtungssituation ausgemessen werden. Dazu werden Informationen zum Belichtungsgrad dieser ersten Aufnahme herangezogen. Diese geben an, welcher Maximalwert einer bestimmten Anzahl an Pixeln erreicht oder überschritten wurde. Es kann auch ein relativer Wert mit Bezug zu einem maximal zulässigen Wert sein, z.B. in Prozent zur Sättigungsgrenze. Informationen zum Belichtungsgrad kann auch das jeweilige Histogramm beinhalten.

Bei AEC- Röntgensystemen mit Scout Shot ist eine schnelle Auswertung der vorbelichteten Aufnahmen zur Bestimmung der vollständigen Röntgenexposition erforderlich, um Bewegungsartefakte zu reduzieren, die durch Bewegungen der Aufnahmegeometrie während der Aufnahmesession hervorgerufen werden können. Bisher erfolgt die Auswertung des Belichtungsgrades des Scout Shots losgelöst vom intraoralen Röntgensensor (im Folgenden auch kurz "sensor " genannt) durch eine Auswerteeinheit, die im Röntgengerät (oder im angeschlossenen Rechner) angeordnet ist. Der bisherige Ablauf ist wie folgt:
1. Sensor erfasst den 1.ten Shot
2. Sensor überträgt den 1.ten Shot ans Röntgengerät
3. Auswerteeinheit analysiert 1. Shot im Röntgengerät
4. Auswerteeinheit legt Belichtungsgrad für den 2.ten Shot fest
5. Sensor erfasst den 2.ten Shot
6. Sensor überträgt den 2.ten Shot ans Röntgengerät
7. Auswerteeinheit berechnet im Röntgengerät aufsummiertes Bild aus den 1.ten Shot und 2.ten Shot

Im Allgemeinen ist das intraorale Röntgengerät mit AEC-Funktion mit einem Rechner oder einer Cloud verbunden. Der in Aufnahmebereitschaft versetzte Intraoral-Röntgensensor erkennt den Start einer Röntgenstrahlenbelichtung und erfasst das Röntgenbild. Nach der Belichtung werden die Röntgenbilddaten aus dem Intraoral-Röntgendetektor ausgelesen und an den Rechner weitergeleitet.

Es wird ferner auf die folgende Dokumente aus dem Stand der Technik verwiesen:
US2003/026387A offenbart die Bestrahlungseinstellung in einem Röntgengerät zur Verwendung bei intraoralen Anwendungen.
EP3399906A1 offenbart ein System und Verfahren zur Anpassung der dentalen Röntgenbestrahlung.

### OFFENBARUNG DER ERFINDUNG

Das Ziel der vorliegenden Erfindung ist einen Intraoral-Röntgensensor mit integrierter AEC-Funktionalität zur Verwendung mit einem Intraoral-Röntgensystem bereitzustellen.

Dieses Ziel wurde durch den intraoralen (IO) Röntgensensor mit integrierter AEC-Funktionalität nach Anspruch 1 erreicht. Die Gegenstände der abhängigen Ansprüche beziehen sich auf Weiterentwicklungen und bevorzugte Ausführungformen.

Gemäß der vorliegenden Erfindung wird die Analyse des Scout Bildes oder Video Streams hinsichtlich des Belichtungsgrades vom IO-Röntgensensor durchgeführt, *nicht* vom Röntgengerät. Dazu kann der Video Stream z.B. als unabhängige Folge von Einzelbildern verwendet werden.

Wird die oben genannte Auswertung in den IO-Röntgensensor (im Nachfolgenden auch kurz "sensor" genannt) verlagert reduziert sich der Datentransfer und die Komplexität auf der Seite des Röntgengerätes. Der Ablauf ist dann z.B. wie folgt
1. Sensor erfasst den 1.ten Shot (Scout Shot);
2. Auswerteeinheit (z.B. eine Exposition Analyse-Einheit des Sensors) analysiert 1. Shot und stellt Informationen zum Belichtungsgrad zusammen;
3. Informationen zum Belichtungsgrad, sowie ggf. Sensor Eigenschaften, werden an Entscheidungseinheit außerhalb des Sensors übertragen;
4. Entscheidungseinheit bestimmt anhand dieser und weiterer Informationen die Belichtungsparameter weiterer Shots fest;
5. Entsprechend dieser Entscheidung erfolgen keine, eine oder weitere Belichtungen, die der Sensor erfasst;
6. Sensor überträgt Einzelaufnahmen und/oder (optional) mindestens eine summierte Aufnahme an das Röntgengerät bzw. PC.

Wesentliches Merkmal der vorliegenden Erfindung ist, dass die dem IO-Röntgensensor zugeordnete Elektronik (z.B. eine Exposition Analyse-Einheit ) in der Lage ist Informationen zum Belichtungsgrad des Scout Bildes oder Video Streams zu erfassen und diese Information dem Röntgengerät mitzuteilen. Dadurch reduziert sich der Initialisierungsaufwand und der Ablauf wird beschleunigt.

Das Röntgengerät, insbesondere die Entscheidungseinheit des Röntgengeräts, verwendet diese Information zum Belichtungsgrad und außerdem sensorspezifische Eigenschaften, vorzugsweise geforderte Bildqualität, Indikation, kV-Anpassung bei Dual Energy, erhöhte Dynamikbereich (HDR), Dosisabhängiges Rauschverhalten, usw., um Belichtungsparameter z.B. Anzahl der Aufnahmen, Belichtungszeit, Röhrenstrom und Röhrenspannung, für die nachfolgende Belichtung festzulegen. Dies könnte eine weitere Belichtung, aber auch mehrere oder keine weitere Belichtung sein.

Im Fall eines Video Streams wird bei Erreichen eines von der Entscheidungseinheit des Röntgengeräts berechneten Wertes die Belichtung vom Röntgengerät vorzugsweise abgebrochen.

Als mögliche Unterbringungsorte für die Auswerteeinheit (z.B. Exposition Analyse-Einheit) bietet sich vorzugsweise der IO-Röntgensensorkopf oder der Röntgensensorstecker an. Alternativ könnte der Sensorkopf mit dem Röntgengerät drahtlos verbunden werden, so dass kein Stecker benötigt wird.

### KURZBESCHREIBUNG DER ZEICHNUNG

In der nachfolgenden Beschreibung wird die vorliegende Erfindung anhand von Ausführungsbeispielen unter Bezugnahme auf die Zeichnung näher erläutert.

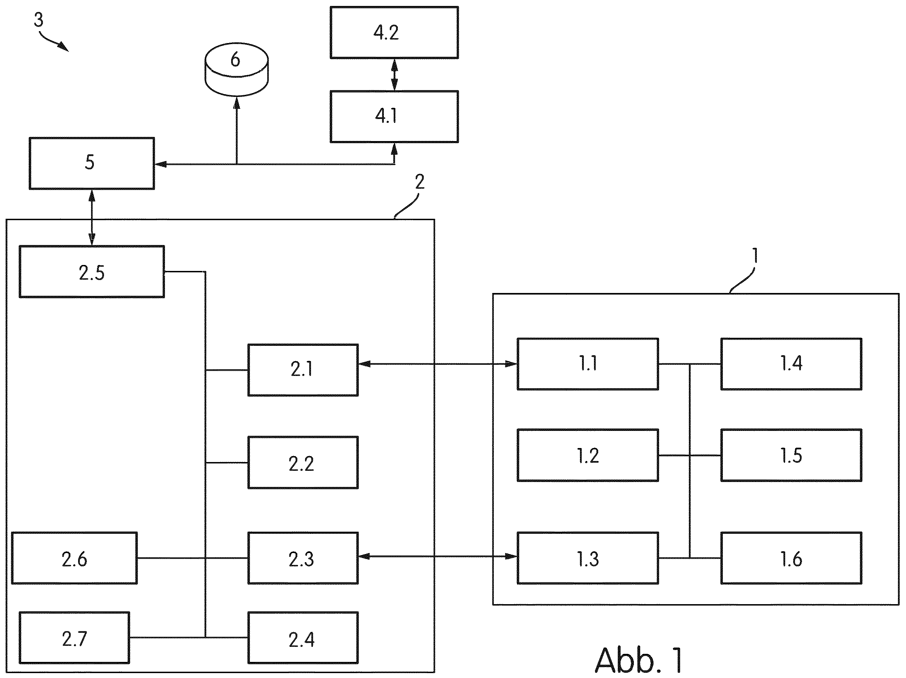

Abb.1 - zeigt eine schematische Darstellung eines Intraoral-Röntgensystems (3) mit einem AEC fähigen IO-Röntgensensor nach einer Ausführungsform der Erfindung.

Die in der Zeichnung gezeigten Referenznummern bezeichnen die unten aufgeführten Elemente, auf die in der nachfolgenden Beschreibung der beispielhaften Ausführungsformen Bezug genommen wird.
- 1.: Intraoral Röntgensensor mit AEC Funktionalität
- 1.1: Kommunikationsschnittstelle
- 1.2: Exposition Analyse-Einheit
- 1.3: Bildgebender Röntgendetektor
- 1.4: Steuerung
- 1.5: Sensorspezifische Eigenschaften
- 1.6: Bildspeicher
- 2.: Röntgengerät
- 2.1: Kommunikationsschnittstelle
- 2.2: Entscheidungs-Einheit
- 2.3: Röntgenstrahler
- 2.4: Steuerung
- 2.5: Kommunikationsschnittstelle
- 2.6: Stromversorgung und Hochspannungsquelle
- 2.7: Benutzerschnittstelle
- 3.: Intraorales Röntgensystem
- 4.1: Rechner
- 4.2: Benutzerschnittstelle und Bildschirm
- 5.: Netzwerk
- 6.: Cloud

Abb.1 zeigt eine schematische Darstellung eines Intraoral-Röntgensystems (3) gemäß eines Ausführungsbeispiels der Erfindung. Das intraorale Röntgensystem (3) hat ein intraorales Röntgengerät (2), das mit einem intraoralen Röntgensensor (1) verbunden ist. Die Verbindung ist vorzugsweise eine Kabelverbindung. Der IO-Röntgensensor (1) bestehet aus einem Sensorkopf (Gehäuse), Kabel und Stecker. Über diesen Stecker ist der IO-Röntgensensor (1) mit dem intraoralen Röntgengerät (2) verbunden. Am IO-Röntgengerät (2) können vorzugsweise gleichzeitig weitere Sensoren (1) mit gleicher AEC Funktionalität über deren Stecker angeschlossen sein. Alternativ kann eine drahtlose Verbindung benutzt werden. Der intraorale Röntgensensor (1) hat eine Kommunikationsschnittstelle (1.1), eine Exposition Analyse-Einheit (1.2), einen bildgebenden Röntgendetektor (1.3), eine Steuerung (1.4), einen Speicher (1.5), der die sensorspezifische Eigenschaften beinhaltet, und einen Bildspeicher (1.6). Die Steuerung (1.4) hat Zugriff auf alle Komponenten des IO-Röntgensensors (1), u.a. auf die Kommunikationsschnittstelle (1.1), die Exposition Analyse-Einheit (1.2), den bildgebenden Röntgendetektor (1.3), den Speicher (1.5), und den Bildspeicher (1.6), um diese zu steuern. Der Bildspeicher (1.6) speichert die vom bildgebenden Röntgendetektor (1.3) empfangenen Bilddaten teilweise oder vollständig. Der Bildspeicher (1.6) ist nützlich, falls sich Pixelbilddaten aus dem bildgebenden Röntgendetektor (1.3) nicht ohne Signalverlust zur Auswertung auslesen lassen, oder ein Signalverlust aus Gründen der Strahlenhygiene nicht akzeptabel ist. Vorzugsweise kann der Röntgensensor (1) mit einer Batterie und einer drahtlosen Kommunikation ausgestattet sein. Dies erlaubt, auf ein Kabel zu verzichten. Eine kabelgebundene Datenkommunikation und/oder Energieversorgung kann auch alternativ berücksichtigt werden. Hier bietet sich als Standard eine USB-Schnittstelle oder Power over Ethernet (PoE) an.

Der intraorale Röntgensensor (1) beinhaltet Komponenten für eine automatische Belichtungskontrolle (AEC) Funktionalität, die im Folgenden naher erklärt wird. Der vom bildgebenden Röntgendetektor (1.3) empfangene Scout Shot oder Scout Video Stream wird von der Exposition Analyse-Einheit (1.2) *lokal im* intraoralen Röntgensensor (1) analysiert, um Informationen zum Belichtungsgrad zu erfassen. Das Analyseergebnis inklusive der Informationen zum Belichtungsgrad wird mittels der Kommunikationsschnittstelle (1.1) an das intraorale Röntgengerät (2) bzw. an ein anderes externes Gerät (z.B. ein Rechner) weitergeleitet. Dazu hat das intraorale Röntgengerät (2) entsprechende Kommunikationsschnittstellen (2.1; 2.5). Das intraorale Röntgengerät (1) hat eine Entscheidungs-Einheit (2.2), die das Analyseergebnis inklusive der erfassten Information zum Belichtungsgrad auswertet und über die Abfolge weiterer Belichtungen insbesondere die Anzahl der Schüsse und deren Dauer entscheidet. Im Fall eines Video Streams kann dies auch während der laufenden Belichtung erfolgen. Bei der Verwendung eines Videostreams erweitert sich der erste Shot und zweite Shot auf die Menge von ersten "m" Shots und zweiten "n" Shots. In diesem Fall kann der Prozess auch mehrfach wiederholt werden. Der IO-Röntgensensor (1) bleibt bis zum Ende der Aufnahme-Session in entsprechender Aufnahmebereitschaft. Das intraorale Röntgengerät (1) hat ferner einen Röntgenstrahler (2.3), eine Stromversorgung und Hochspannungsquelle (2.6) und eine Steuerung (2.4). Die Steuerung (2.4) hat u.a. auch Zugriff auf den IO-Röntgensensor (1). Das intraorale Röntgengerät (1) ist vorzugsweise insbesondere ein Kleinbildröntgengerät für dentale Behandlungen. Die Entscheidungseinheit (2.2) ist ferner ausgebildet, die sensorspezifischen Eigenschaften (1.5) und optional die vom Anwender vorgegebenen Bildqualitätsparameter zu berücksichtigen. Die sensorspezifischen Eigenschaften (1.5) umfassen Angaben u.a. zu Sensor-Dimensionen, zu lokalen Pixelfehlern, zum Dosis/Signal-Verhalten und zum maximalen Sättigungspegel, ab dessen Überschreiten Sättigungseffekte eintreten, die keine oder eingeschränkte Digitalisierung des analogen Belichtungssignals erlauben. Die Bildqualitätsparameter können vom Benutzer direkt oder indirekt durch eine Benutzerschnittstelle (2.7) am Röntgengerät (2) eingegeben werden. Eine indirekte Vorgabe kann über die ärztliche Indikation (z.B. Klärung von Verdacht auf Karies, Paradentose, Wurzelverlauf) erfolgen, die vom intraoralen Röntgensystem (3) mit der Entscheidungseinheit (2.2) in Belichtungsparameter umgesetzt wird, z.B. maximale Belichtung, Dual Energy etc.. Alternativ kann dies auch über eine Benutzerschnittstelle (4.2) eines Rechners (4.1) geschehen, der am intraoralen Röntgengerät (2) angeschlossen ist. Das intraorale Röntgengerät (2) ist mit seiner Kommunikationsschnittstelle (2.5) und vorzugsweise über ein Netzwerk (5) mit einem Rechner (4.1) verbunden. Das intraorale Röntgengerät (2) und der Rechner (4.1) können auch eine Verbindung zu einer Cloud (6) haben. Anstelle, dass der IO-Röntgensensor (1) oder das IO Röntgengerät (2) bildverarbeitende Funktionen ausführt, kann dies in der Cloud (6) oder durch den PC (4.1) erfolgen. Das empfangene Rohbildmaterial wird zu einem ersten Rohbild aufgearbeitet, um sensor- und aufnahmespezifische Unzulänglichkeiten zu kompensieren. Diese sind: Gain, Blemish- und DC-Korrektur (klassisch), Dynamik-Erweiterung (HDR), Bewegungsartefakt-Kompensation (Anti-Shake).

## Patentansprüche

1. Intraoral-Röntgensensor (1) zur Verwendung mit einem Intraoral-Röntgensystem (3) mit einer Automatischen Belichtungskontrolle (AEC) Funktionalität, wobei der Intraoral-Röntgensensor (1) eine Exposition Analyse-Einheit (1.2), einen bildgebenden Röntgendetektor (1.3), einen Speicher (1.5), der sensorspezifische Eigenschaften beinhaltet, und eine Kommunikationsschnittstelle (1.1) aufweist, wobei ein vom bildgebenden Röntgendetektor (1.3) empfangener Scout Shot oder Scout Video Stream von der Exposition Analyse-Einheit (1.2) im Intraoral-Röntgensensor (1) analysiert wird, um Informationen zum Belichtungsgrad des Scout Bildes oder Video Streams zu erfassen und diese, sowie die sensorspezifischen Eigenschaften, mittels der Kommunikationsschnittstelle (1.1) an eine zum Intraoral-Röntgensensor (1) extern angeordnete Entscheidungseinheit (2.2) des Intraoral-Röntgensystems (3) zur Auswertung und Entscheidung weiterer Belichtungen weiterzuleiten, wobei die sensorspezifischen Eigenschaften (1.5) Angaben umfassen zu Sensor-Dimensionen, zu lokalen Pixelfehlern, zum Dosis/Signal-Verhalten und zum maximalen Sättigungspegel, ab dessen Überschreiten Sättigungseffekte eintreten, die keine oder eingeschränkte Digitalisierung des analogen Belichtungssignals erlauben.

2. Intraoral-Röntgensensor (1) nach Anspruch 1, wobei dieser einen Bildspeicher (1.6) umfasst, der geeignet ist, vom bildgebenden Röntgendetektor (1.3) empfangene Bilddaten teilweise oder vollständig zu speichern.

3. Intraoral-Röntgensystem (3), umfassend einen Intraoral-Röntgensensor (1) nach Anspruch 1 oder 2, und ein Intraoral-Röntgengerät (2) mit einer Automatischen Belichtungskontrolle (AEC) Funktionalität, wobei das Intraoral- Röntgengerät (2) und der Intraoral-Röntgensensor (1) mittels jeweiligen Kommunikationsschnittstellen (1.1; 2.1) verbunden sind, und wobei das Intraoral- Röntgengerät (2) eine Entscheidungseinheit (2.2) beinhaltet zur Auswertung der Informationen zum Belichtungsgrad, und zur Entscheidung über die Abfolge weiterer Belichtungen insbesondere die Anzahl der Schüsse und deren Dauer; wobei das Intraoral-Röntgengerät (2) eine Steuerung (2.4) aufweist, welche Zugriff auf den IO-Röntgensensor (1) hat, und die Entscheidungseinheit (2.2) ferner ausgebildet ist, sensorspezifische Eigenschaften (1.5) zu berücksichtigen.

## Claims

1. Intraoral X-ray sensor (1) for use with an intraoral X-ray system (3) having an automatic exposure control (AEC) functionality, wherein the intraoral X-ray sensor (1) has an exposure analysis unit (1.2), an imaging X-ray detector (1.3), a memory (1.5) with sensor-specific properties, and a communication interface (1.1),
wherein a scout shot or scout video stream received by the imaging X-ray detector (1.3) is analyzed by the exposure analysis unit (1.2) in the intraoral X-ray sensor (1) to record information on the degree of exposure of the scout image or video stream and to forward this, as well as the sensor-specific properties, via the communication interface (1.1), to a decision unit (2.2) of the intraoral X-ray system (3) arranged externally to the intraoral X-ray sensor (1) for evaluation and decision on further exposures,
wherein the sensor-specific properties (1.5) comprise information on sensor dimensions, local pixel errors, dose/signal behavior and the maximum saturation level, above which saturation effects occur that allow no or limited digitization of the analog exposure signal.

2. Intraoral X-ray sensor (1) according to claim 1, wherein it comprises an image memory (1.6) which is suitable for partially or completely storing image data received from the imaging X-ray detector (1.3).

3. Intraoral X-ray system (3), comprising an intraoral X-ray sensor (1) according to claim 1 or 2, and an intraoral X-ray device (2) with an automatic exposure control (AEC) functionality, wherein the intraoral X-ray device (2) and the intraoral X-ray sensor (1) are connected by means of respective communication interfaces (1.1; 2.1), and wherein the intraoral X-ray device (2) includes a decision unit (2.2) for evaluating the information on the degree of exposure and for deciding on the sequence of further exposures, in particular the number of shots and their duration, wherein the intraoral X-ray device (2) has a controller (2.4) which has access to the IO X-ray sensor (1), and the decision unit (2.2) is further designed to take sensor-specific properties (1.5) into account.

## Revendications

1. Capteur radiographique intra-oral (1) destiné à être utilisé avec un système radiographique intra-oral (3) ayant une fonctionnalité de contrôle automatique de l'exposition (AEC), le capteur radiographique intra-oral (1) comprenant une unité d'analyse de l'exposition (1.2), un détecteur de rayons X d'imagerie (1.3), une mémoire (1.5) qui contient des propriétés spécifiques au capteur, et une interface de communication (1.1), un scout shot ou un flux scout video reçu par le détecteur de rayons X d'imagerie (1.3) transmis par l'unité d'analyse d'exposition (1.2) dans le capteur radiographique intra-oral (1) étant analysée afin de saisir des informations sur le degré d'exposition de l'image scout ou du flux vidéo et de les transmettre, ainsi que les propriétés spécifiques au capteur, au moyen de l'interface de communication (1.1) à une unité de décision (2.2) du système radiographique intra-oral (3) pour l'évaluation et la décision d'autres expositions, les propriétés spécifiques au capteur (1.5) comprenant des indications sur les dimensions du capteur, sur les erreurs locales de pixels, sur le comportement dose/signal et sur le niveau de saturation maximal à partir duquel des effets de saturation se produisent, qui ne permettent aucune numérisation ou une numérisation limitée du signal d'exposition analogique.

2. Capteur radiographique intra-oral (1) selon la revendication 1, celui-ci comprenant une mémoire d'image (1.6) qui est adaptée pour stocker partiellement ou totalement des données d'image reçues du détecteur de rayons X d'imagerie (1.3).

3. Système radiographique intra-oral (3), comprenant un capteur radiographique intra-oral (1) selon la revendication 1 ou 2, et un appareil radiographique intra-oral (2) avec une fonctionnalité de contrôle automatique de l'exposition (AEC), l'appareil radiographique intra-oral (2) et le capteur radiographique intra-oral (1) étant reliés par des interfaces de communication respectives (1.1;2.1), et l'appareil radiographique intra-oral (2) comprenant une unité de décision (2.2) pour l'évaluation des informations sur le degré d'exposition, et pour la décision sur la séquence d'autres expositions, en particulier le nombre de tirs et leur durée ;
l'appareil radiographique intra-oral (2) présentant une commande (2.4) qui a accès au capteur radiographique IO (1), et l'unité de décision (2.2) étant en outre conçue pour prendre en compte des propriétés (1.5) spécifiques au capteur.
